# EUROPEAN PATENT APPLICATION

(11) **EP 2 407 162 A1**
(43) Date of publication of application: **18.01.2012**
(21) Application number: 10750926.7
(22) Date of filing: 12.03.2010
(51) Int. Cl.: A61K 31/198, A61K 9/16, A61P 1/00, A61P 1/04

(54) **COMPOSITION FOR ORAL ADMINISTRATION**

(30) Priority: 13.03.2009 JP 2009062016
(71) Applicant: Ajinomoto Co., Inc., Tokyo 104-8315 (JP); National University Corporation Gunma University, Maebashi-shi, Gunma 371-8510 (JP)
(72) Inventor: FUJITA, Shinichi, Kawasaki-shi Kanagawa 210-8681 (JP); HANZAWA, Yoshiaki, Tokyo 104-0042 (JP); YASA, Noriko, Tokyo 104-0042 (JP); MATSUEDA, Hiroyuki, Tokyo 104-0042 (JP); KIHARA, Hideaki, Tokyo 104-0042 (JP); KUSANO, Motoyasu, Maebashi-shi Gunma 371-8511 (JP); KAWAMURA, Osamu, Maebashi-shi Gunma 371-8511 (JP); SHIMOYAMA, Yasuyuki, Maebashi-shi Gunma 371-8511 (JP); HOSAKA, Hiroko, Maebashi-shi Gunma 371-8511 (JP); ZAI, Hiroaki, Maebashi-shi Gunma 371-8511 (JP); NAGOSHI, Atsuto, Maebashi-shi Gunma 371-8511 (JP)
(74) Representative: Nicholls, Kathryn Margaret
(86) International application number: PCT/JP2010/054236
(87) International publication number: WO 2010/104175

(57) **Abstract**

The present invention provides an oral composition which can more effectively prevent or improve functional gastrointestinal disorders, particularly upper gastrointestinal dysfunction, specifically functional dyspepsia, and is highly safe and suitable for long-term ingestion.

The oral composition of the present invention contains sodium glutamate and arginine hydrochloride at a molar ratio of 1:1. The aforementioned composition can be provided as an agent for the prophylaxis or improvement of functional gastrointestinal disorder, and can also be provided in a food or drink.

## Description

### Technical Field

The present invention relates to an oral composition having a prophylactic or improving effect on functional gastrointestinal disorders, and particularly relates to an oral composition effective for the prophylaxis or improvement of upper gastrointestinal dysfunctions such as functional dyspepsia and esophageal reflux, specifically, postprandial complaints such as postprandial fullness, early satiation and the like, functional dyspepsia showing epigastric pain and the like.

### Background Art

Functional dyspepsia (FD) is a symptom derived from the upper gastrointestinal tract, which shows no pathological condition or no clear pathological condition, and even if it shows pathological condition, findings sufficient to explain the clinical condition are not obtained. According to the Rome III standard, which is the diagnostic standard of functional gastrointestinal disorder, FD is diagnosed when at least one of the symptoms relating to postprandial distress syndromes (PDS) such as postprandial fullness and early satiation, and symptoms relating to epigastric pain syndromes (EPS) such as epigastric pain and epigastric burning is chronically observed (strictly not less than 3 months), and structural diseases are excluded.

In addition, gastro-esophageal reflux disease (GERD) means refluxing of stomach content to esophagus, and refers to functional disorders of the lower esophagus sphincter. The factors involved in the functions of the gastroesophageal junction include the original sphincter contraction pressure, angle of esophagocardial junction, movement of the diaphragm and gravity (when patients are rising to their full height). GERD sometimes causes esophagitis.

In Japan, each case of gastrointestinal dysfunction, particularly upper gastrointestinal dysfunction (Functional gastrointestinal disorders; FGIDs), such as the above-mentioned functional dyspepsia and gastro-esophageal reflux disease has been diagnosed as "upper abdominal gastrointestinal complaint associated with chronic gastritis" irrespective of organic findings and, in clinical situations, diagnosed conventionally as "gastritis" or "chronic gastritis".

Even in cases where an organic disease (erosive esophagitis, peptic ulcer, acute gastritis, gastrointestinal cancer etc.) is observed, abdominal pain, discomfort, postprandial heavy stomach, nausea·vomiting and the like are also found. Therefore, there is an urgent need for the improvement of such discomfortable feeling to ensure better quality of life (QOL) of patients. When functional dyspepsia is joined with lower abdominal indefinite complaint such as defecation difficulty due to constipation, unrelieved feeling after defecation, abdominal pain, feeling of fullness of the abdomen and the like, about 30%-about 50% of the total population of Japan is assumed to have experienced some gastrointestinal indefinite complaint. While the onset of such indefinite complaints of the gastrointestinal tract is considered to be under influence by sex, age, stress or obesity, the only suggested mechanism of its onset is degraded gastrointestinal motility function, and has not been completely clarified.

In addition, many of the patients with a progressive degenerative disease of the brain such as Parkinson's disease, Huntington chorea, olivopontocerebellar atrophy and the like, or cerebrovascular disorder also develop gastrointestinal motility dysfunction. However, it is considered that many of these patients cannot report indefinite complaint by themselves due to logopathy, disturbance of consciousness and the like. Thus, it is important for the improvement of QOL of the patients to provide a care that removes abdominal uncomfortable symptoms simultaneously with a care of organic dysfunction.

Dopamine D₂ receptor antagonists, 5-HT₄ receptor agonists and the like have heretofore been used for the treatment of functional dyspepsia and gastro-esophageal reflux disease. For example, cisapride and metoclopramide increase release of acetylcholine by antagonizing dopamine D₂ receptor and stimulating 5-HT₄ receptor present in the gastrointestinal tract intramural plexus, which promotes gastric motility and stomach evacuation to improve uncomfortable abdominal symptoms such as feeling of fullness of the abdomen and the like. However, cisapride is known to cause side effects including serious side effects such as prolongation of QT (time from the start of ventricular depolarization to completion of ventricular repolarization), ventricular arrhythmia, Parkinson symptom (tremor, muscular rigidity, akinesia, short-stepped gait) and the like, and side effect of extrapyramidal symptoms caused by the action on dopamine D₂ receptors in the central nervous system (ataxia, spasm in muscle and limbs, torticollis). In addition, metoclopramide also causes side effects such as convulsion, tardive dyskinesia and the like, as well as extrapyramidal symptom and the like. Besides these, mosapride citrate and the like are also used. However, the effect is not sufficient in some cases, and side effects such as diarrhea and the like sometimes develop. Furthermore, H₂ receptor antagonist, proton pump inhibitor and the like are also used. However, its safety in long-term ingestion is unclear, and long-term ingestion of proton pump inhibitor is reported to increase the risk of bone fracture and the like.

On the other hand, glutamic acid has been reported to act as an agent for the prophylaxis or improvement of functional gastrointestinal disorders. Furthermore, it has been disclosed that an arginine salt of glutamic acid is also useful as an agent for the prophylaxis or improvement of gastrointestinal disorders (patent document 1). In addition, the present applicant has filed a patent application for a combination of glutamic acid or a salt thereof, and arginine or a salt thereof for effective improvement of functional gastrointestinal disorders such as functional dyspepsia and the like.

### [Document List]

### [patent document]

patent document 1: WO2006/030980

### [SUMMARY OF THE INVENTION]

### Problems to be Solved by the Invention

Thus, the present invention aims to provide an oral composition which can more effectively prevent or improve functional gastrointestinal disorders, particularly upper gastrointestinal dysfunction, specifically functional dyspepsia, and is highly safe and suitable for long-term ingestion.

### Means of Solving the Problems

In an attempt to solve the above-mentioned problems, the present inventors have conducted intensive studies and found that a prophylactic or improving effect on functional gastrointestinal disorders, particularly a superior prophylactic or improving effect on upper gastrointestinal dysfunction such as functional dyspepsia and the like, can be obtained by using sodium glutamate and arginine hydrochloride at a molar ratio of 1:1, which resulted in the completion of the present invention.

Accordingly, the present invention relates to the following [1] - [16].
[1] An oral composition comprising sodium glutamate and arginine hydrochloride at a molar ratio of 1:1.
[2] The oral composition of the above-mentioned [1], wherein sodium glutamate and arginine hydrochloride are contained in a total amount of 0.05 g - 10 g.
[3] The oral composition of the above-mentioned [1], wherein sodium glutamate and arginine hydrochloride are contained in a total amount of 0.1 g - 5 g.
[4] The oral composition of the above-mentioned [1], wherein sodium glutamate and arginine hydrochloride are contained in a total amount of 0.3 g - 3 g.
[5] The oral composition of any of the above-mentioned [1] - [4], wherein a total amount of sodium glutamate and arginine hydrochloride to be ingested per day for an adult is 0.3 g - 10 g.
[6] The oral composition of any of the above-mentioned [1] - [5], wherein the amount to be ingested per day is taken in one to 3 portions.
[7] The oral composition of any of the above-mentioned [1] - [6] for consecutive ingestion for 7 days or longer.
[8] The oral composition of any of the above-mentioned [1] - [7], which is granule.
[9] The oral composition of any of the above-mentioned [1] - [8], which is an oral agent for the prophylaxis or improvement of a functional gastrointestinal disorder.
[10] The oral composition of the above-mentioned [9], wherein the functional gastrointestinal disorder is upper gastrointestinal dysfunction.
[11] The oral composition of the above-mentioned [10], wherein the upper gastrointestinal dysfunction is functional dyspepsia.
[12] A food or drink containing the oral composition of any of the above-mentioned [1] - [11].
[13] A method for the prophylaxis or improvement of a functional gastrointestinal disorder, comprising orally administering the oral composition of the above-mentioned [1] in an amount sufficient for the prophylaxis or improvement of functional gastrointestinal disorder to a patient with a functional gastrointestinal disorder.
[14] A method for the prophylaxis or improvement of an upper gastrointestinal dysfunction, comprising orally administering the oral composition of the above-mentioned [1] in an amount sufficient for the prophylaxis or improvement of upper gastrointestinal dysfunction to a patient with an upper gastrointestinal dysfunction.
[15] A method for the prophylaxis or improvement of functional dyspepsia, comprising orally administering the oral composition of the above-mentioned [1] in an amount sufficient for the prophylaxis or improvement of functional dyspepsia to a patient with functional dyspepsia.
[16] A commercial package comprising the oral composition of any of the above-mentioned [1] - [12] and a document stating that said oral composition can or should be used for the prophylaxis or improvement of a functional gastrointestinal disorder.

### Effect of the Invention

The present invention can provide an oral composition capable of effectively improving gastrointestinal dysfunction associated with functional degradation of the gastrointestinal tract. In addition, since the oral composition of the present invention is highly safe and suitable for a long-term ingestion, it is also effective for the prevention of the development of gastrointestinal dysfunction. The oral composition of the present invention is particularly effective for the prophylaxis or improvement of upper gastrointestinal dysfunction, specifically various symptoms of functional dyspepsia. Furthermore, the oral composition of the present invention can reduce uncomfortable feeling of patients showing various symptoms of gastrointestinal dysfunction such as functional dyspepsia and the like, for a long time, and can enhance QOL of the patients.

### [Description of Embodiments]

The oral composition of the present invention characteristically contains sodium glutamate and arginine hydrochloride at a molar ratio of 1:1, prevents or improves reproducible functional gastrointestinal disorder that decreases QOL of patients, and can be effectively used as an oral agent for the prophylaxis or improvement of a functional gastrointestinal disorder.

The "functional gastrointestinal disorder" in the present invention refers to a state showing decreased function of gastrointestinal tract even in the absence of clear organic changes, and includes any functional decrease in the gastrointestinal tract, that is, pharynx, esophagus, stomach, small intestine (duodenum, jejunum, ileum) and large intestine. In Rome III, the definition is divided into A to H, which are functional esophagus disorder, functional duodenal disorder, functional bowel disorder, functional abdominal pain syndrome, functional gallbladder and sphincter of Oddi disorder, functional anorectal disorder, infant and toddler functional disorder, and child and adolescent functional disorder.

The oral composition of the present invention is particularly effective for the prophylaxis or improvement of upper gastrointestinal dysfunction. Here, the "upper gastrointestinal" means pharynx, esophagus, stomach and duodenum, and examples of the "upper gastrointestinal dysfunction" include the aforementioned functional dyspepsia and gastro-esophageal reflux disease. The oral composition of the present invention is specifically effective for the prophylaxis or improvement of functional dyspepsia.

Specific symptoms of functional gastrointestinal disorders that can be prevented or improved by the oral composition of the present invention include representative upper gastrointestinal indefinite complaints such as sick feeling, vomiting, nausea, heartburn, feeling of fullness in the abdomen, heavy stomach, belching, chest writhing, chest pain, gastric discomfort, anorexia and the like, lower gastrointestinal indefinite complaints such as abdominal pain, constipation, diarrhea and the like, and related complaints such as breathlessness, feeling of smothering, low incentive, pharyngeal obstruction · feeling of foreign substance ("*baikakuki*" in Chinese medicine), easy fatigability, stiff neck, myotonia, dry mouth · thirst, tachypnea, burning sensation · cold sensation of extremities, difficulty in concentration, impatience, sleep disorder, headache, general malaise, palpitation, night sweat, anxiety, dizziness, vertigo, burning sensation, hot flash, sweating, abdominal pain, constipation, depression and the like. The oral composition of the present invention is effective for the prophylaxis or improvement of upper gastrointestinal indefinite complaint from among those, and is particularly effective for the prophylaxis or improvement of postprandial complaints such as postprandial fullness and early satiation, and epigastric pains such as epigastric pain and epigastric burning.

In the oral composition of the present invention, any of an L form, a D form and a DL form can be used as sodium glutamate or arginine hydrochloride, and an L form is used most preferably. In addition, as sodium glutamate and arginine hydrochloride, any of the amino acids obtained by extraction from animals and plants etc. containing them, and purification thereof, and the amino acids obtained by a chemical synthesis method, a fermentation method or a gene recombination method may be used, and a commercially available product can also be purchased from Ajinomoto Co., Inc., Sigma-Aldrich Corp. and the like.

The oral composition of the present invention contains sodium glutamate and arginine hydrochloride at a molar ratio of 1:1. The molar ratio is set to 1:1 in an attempt to reduce stimulation to the stomach mucosa by decreasing the influence of pH when adding glutamic acid (acidic amino acid) and arginine (basic amino acid), each in a salt form. The content of sodium glutamate and arginine hydrochloride in the composition is preferably 0.05 g - 10 g, more preferably 0.1 g - 5 g, particularly preferably 0.3 g - 3 g, in total amounts. This is because, the sodium glutamate and arginine hydrochloride in the present invention shows effect in a dose-dependent manner, and on the other hand, the more content of the sodium glutamate and arginine hydrochloride is difficult for oral ingestion in view of compliance.

In addition, in the oral composition of the present invention, the amount of sodium glutamate and arginine hydrochloride to be ingested varies depending on the age, body weight and sex of the subject who ingests them, diet, symptom and level of functional gastrointestinal disorder, risk of inducing gastrointestinal dysfunction, presence or absence and level of organic disorder in the gastrointestinal tract and the like, and is suitably 0.3 g - 10 g per day for an adult. This may be ingested at once or in several divided portions. When the oral composition of the present invention is ingested particularly for a long time and the like, one to 3 times of ingestion per day is preferable for preventing missing doses.

Since functional gastrointestinal disorder is often developed chronically, the oral composition of the present invention is preferably ingested consecutively for a long time for the prophylaxis or improvement of the disease. To afford a clear prophylactic or improving effect, it is preferably ingested consecutively for 7 days or longer.

The oral composition of the present invention can also be ingested to animals other than human. Examples of the animals other than human include domestic animals and poultries such as bovine, horse, swine, sheep, chicken and the like, pet animals such as dog, cat, rabbit and the like, experimental animals such as mouse, rat etc. and the like.

As the oral composition of the present invention, effective amounts of sodium glutamate and arginine hydrochloride may be directly ingested, or they may be formulated with a pharmaceutically acceptable carrier for pharmaceutical products and provided as an oral agent for the prophylaxis or improvement of a functional gastrointestinal disorder. In the present invention, any dosage form can be employed as long as it is suitable for oral ingestion and, for example, liquid preparations such as elixir, suspension, syrup, emulsion, ampoule and the like; solid preparations such as gel, gum, drop, powder, granule, pill, tablet (including sugar-coated tablet, film-coated tablet), capsule, package agent, etc. and the like can be produced. In addition, sustained-release preparations such as gel-coated preparation, multi-coated preparation and the like, localized release preparations such as pyloric part rupture preparation etc. and the like can be also used. In the present invention, granule is preferable from the aspects of easy formulation, cost, preparation stability, easy ingestion and the like.

Examples of the pharmaceutically acceptable carrier for pharmaceutical products usable in the present invention include liquid carriers such as water, alcohol, emulsion and the like; solid carriers such as gel, powder and the like; endoplasmic reticula such as microcapsule, liposome etc. and the like, and various pharmaceutically acceptable additives can be added. Examples of the various additives include excipients such as cellulose (crystalline cellulose, hydroxypropylcellulose and the like) and derivatives thereof, starch (wheat starch, cornstarch, sodium carboxymethyl starch, dextrin and the like) and derivatives thereof, natural polymer compounds (gum arabic, sodium alginate and the like), sugar (glucose, maltose, sorbitol, maltitol, mannitol and the like) and derivatives thereof, inorganic salts (sodium chloride, calcium carbonate, magnesium silicate and the like) and the like; binders such as guar gum, synthesis aluminum silicate, stearic acid, polymer polyvinylpyrrolidone, lactose and the like; lubricants such as talc, magnesium stearate, polyethylene glycol 6000 and the like; disintegrants such as adipic acid, calcium stearate, sucrose and the like; surfactants such as sucrose ester of fatty acid, soybean lecithin, polyoxyethylene hydrogenated castor oil, polyoxyethylene monostearate and the like; thickeners such as sodium carboxymethylcellulose, carboxyvinyl polymer, xanthan gum, gelatin and the like; coating agents such as ethyl acrylate·methyl methacrylate copolymer dispersion, caramel, Carnauba wax, shellac, sucrose, pullulan and the like; pH adjusters such as citric acid, sodium citrate, acetic acid, sodium acetate, sodium hydroxide and the like; antioxidants such as ascorbic acid, tocopherol acetate, natural vitamin E, propyl gallate and the like; flavoring agents such as aspartame, licorice extract, saccharin and the like; preservatives such as sodium benzoate, sodium edetate, sorbic acid, sodium sorbate, methyl paraoxybenzoate, butyl paraoxybenzoate and the like; colorants such as ferric oxide red, yellow iron oxide, black iron oxide, carmine, Food Color blue No.1, Food Color yellow No. 4, Food Color Yellow No. 4 aluminum lake, Food Color Red No. 2, sodium copper chlorophyllin etc. and the like.

The oral composition of the present invention may be concurrently used with other drugs. Examples of such drug include H₂ receptor antagonists such as cimetidine, ranitidine, famotidine, nizatidine and the like; proton pump inhibitors such as omeprazole, lansoprazole, rabeprazole and the like; 5-HT₄ receptor agonists such as mosapride citrate and the like; dopamine D₂ antagonists such as metoclopramide, domperidone and the like; muscarinic receptor antagonists such as tiquizium bromide, pirenzepine hydrochloride and the like; antigastrin drugs such as proglumide, urogastrone and the like; anticholinergic agents such as piperidolate hydrochloride, propantheline bromide, scopolamine butylbromide and the like; belladonna alkaloid such as scopolia extract, atropine sulfate and the like; prostaglandin preparations such as ornoprostil, enprostil, misoprostol and the like; defensive factor enhancers such as teprenone, plaunotol, rebamipide, methylmethionine sulfonium chloride, sucralfate, polaprezinc, sodium azulene sulfonate, egualen sodium, aldioxa, aceglutamide aluminum, cetraxate hydrochloride, licorice extract, sofalcone, gefarnate, sodium alginate, troxipide, benexate hydrochloride betadex, irsogladine maleate, ecabet sodium and the like; steroids such as mesalazine, betamethasone sodium phosphate and prednisolone sodium phosphate; therapeutic drugs for inflammatory bowel diseases such as infliximab etc. and the like. One or more kinds of these can be selected and used.

Furthermore, the oral composition of the present invention can also be provided as it is or in the form of, for example, liquid, emulsion, jelly, gum, powder, granule, sheet, capsule, tablet and the like together with additives generally used for food and drink as, for example, food and drink products such as food with health claims (food for specified health uses and food with nutrient function claims), nutrition aid food, other health food and the like. Examples of the above-mentioned additive include sweetener, colorant, preservative, thickening stabilizer, antioxidant, color former, bleach, fungicide, gum base, bittering agent, enzyme, gloss agent, acidulant, seasoning, emulsifier, enhancement agent, agent for production, flavor, spice extract and the like. In addition, the oral composition of the present invention can also be ingested with a meal, by adding the composition to existing foods such as drink, beverage water, yoghurt, jelly, milk beverage and the like.

When a food or drink containing the oral composition of the present invention is to be provided, the content of sodium glutamate and arginine hydrochloride per a food or drink composition is suitably 0.05 g - 10 g, more preferably 0.1 g - 5 g, particularly preferably 0.3 g - 3 g, in the total amount of these. The amount thereof to be ingested per day by an adult is 0.3 g - 10 g in the total amount of sodium glutamate and arginine hydrochloride, which may be ingested in one to several portions per day.

When a food or drink containing the oral composition of the present invention is to be provided, a package form for one ingestion amount unit containing sodium glutamate and arginine hydrochloride in the above-mentioned amount to be ingested once can be employed. The "package form for one ingestion amount unit" is used when the amount of food and drink to be ingested per meal is determined in advance. Examples thereof include a package of a given amount using a container such as pack, bottle, box and the like in case of drinks, candy, gum, jelly, purine, yogurt and the like, and an individual package of a given amount using pack, bag and the like in case of foods in the form of granule, powder, slurry and the like. Particularly, when the foods and drinks are food with health claims such as food for specified health uses, food with nutrient function claims and the like, nutrition aid food, the other health foods and the like, a form wherein sodium glutamate and arginine hydrochloride is packed in a unit amount to be ingested per meal, a form wherein sodium glutamate and arginine hydrochloride is suspended or dissolved to give a drink, which is packed in a bottle etc. for a single consumption and the like can be mentioned. One ingestion unit amount of sodium glutamate and arginine hydrochloride is suitably 0.1 g - 10 g in a total amount of these, in view of the above-mentioned amount per day for an adult.

The present invention moreover provides a method for the prophylaxis or improvement of functional gastrointestinal disorder, comprising orally administering the oral composition of the present invention to patients showing functional gastrointestinal disorder in an amount sufficient to prevent or improve the functional gastrointestinal disorder. While the "amount sufficient to prevent or improve functional gastrointestinal disorder" varies depending on the age, sex and body weight of patients, symptom and level of functional gastrointestinal disorder and the like, the total amount of sodium glutamate and arginine hydrochloride is generally 0.3 g - 10 g per day for an adult. The aforementioned amount is preferably administered orally in 1 to 3 portions per day. When a functional gastrointestinal disorder is observed chronically, a consecutive administration for 7 days or longer is preferable.

The above-mentioned method for the prophylaxis or improvement of a functional gastrointestinal disorder of the present invention is particularly useful as a method for the prophylaxis or improvement of upper gastrointestinal dysfunction. As the upper gastrointestinal dysfunction, functional dyspepsia can be particularly mentioned as an applicable disease.

Moreover, the present invention can provide a commercial package, which contains the oral composition of the present invention, and a document describing that the oral composition can or should be used for the prevention or improvement of a functional gastrointestinal disorder. Examples of the commercial package of the present invention include a pharmaceutical package containing the oral composition of the present invention, which is an oral agent for the prophylaxis or improvement of a functional gastrointestinal disorder, and the aforementioned document, a food or drink package containing a food or drink containing the oral composition of the present invention, and the aforementioned document, and the like. The aforementioned document describes that the oral composition of the present invention is effective for the prophylaxis or improvement of a functional gastrointestinal disorder, as well as explanation of, for example, specific cases of functional gastrointestinal disorders to be applied for, a target for administration or ingestion, a dose to be taken or ingested at one time, ingestion frequency per day, a method of taking or ingesting and the like.

### Examples

The present invention is explained in more detail by referring to the Examples.

### [Example 1]

For one time ingestion, L-sodium glutamate (0.057 g), L-arginine hydrochloride (0.067 g) and partially gelatinized starch (1.116 g) were each pulverized in a grinding machine and mixed. Ethanol was added and the mixture was kneaded in a kneader, and granulated by an extrusion-granulator to give granules. The amounts of L-sodium glutamate and L-arginine hydrochloride for one time ingestion were each 0.3 mmol (total amount of ingestion 0.124 g).

### [Example 2]

For one time ingestion, L-sodium glutamate (0.57 g) and L-arginine hydrochloride (0.67 g) were each pulverized in a grinding machine and mixed. Ethanol was added and the mixture was kneaded in a kneader, and granulated by an extrusion-granulator to give granules. The amounts of L-sodium glutamate and L-arginine hydrochloride for one time ingestion were 3.4 mmol and 3.2 mmol, respectively (total amount of ingestion 1.24 g).

### [Comparative Example]

For one time ingestion, cornstarch (1.24 g) was pulverized in a grinding machine. Ethanol was added and the mixture was kneaded in a kneader, and granulated by an extrusion-granulator to give granules.

### [Experimental Example]

The granules of Examples 1 and 2 of the present invention and the granules of Comparative Example were subjected to a clinical test with functional dyspepsia patients. The clinical test was performed using 20 to 65-year-old patients as test subjects who satisfy diagnostic standard of functional dyspepsia, developed upper gastrointestinal symptoms such as postprandial fullness, early satiation, and epigastric pain or epigastric burning of a moderate level or higher 6 months or more ago, and had been experiencing such symptoms for the previous 3 months, even in the absence of organic changes by endoscopy. The test subjects were divided into 3 groups, and each group ingested granules of Example 1, Example 2 and Comparative Example by double-blind. The subjects ingested the granules of the Examples and Comparative Example three times a day for 14 days immediately before meal, and the symptoms of upper gastrointestinal tract were evaluated every day in the patient diary. The number of test subjects of each group was 10 for the granule ingestion group of Example 1, 9 for the granule ingestion group of Example 2, and 10 for the granule ingestion group of Comparative Example.

As the upper gastrointestinal symptoms, 8 items shown below were recited, and each item was scored by an integer against 10 as a full mark, where "0: no symptom" and "10: severe symptom". Of the following symptoms of upper gastrointestinal tract, items 1 - 3 relate to postprandial distress syndrome (PDS) and items 4 - 6 relate to epigastric pain syndrome (EPS), according to the Rome III standard.

### [Symptoms of upper gastrointestinal tract]

1. stomach is felt like swelling after eating
2. stomach is full resulting in leftover
3. stomach is felt heavy
4. discomfort in epigastric region is felt
5. feel pain in epigastric region
6. feel epigastric burning
7. feel sick and feel like vomiting
8. chest region is felt burning

For the clinical test results, the scores of each of the above-mentioned upper gastrointestinal symptoms of each test subject were totalized every day for PDS (1 - 3), EPS (4 - 6), PDS+EPS (1 - 6), all symptoms (1 - 8), and average scores of each ingestion group are shown in Table 1.

**Table 1**

| symptom | ingestion group | before ingestion | days after ingestion (days) | | | | | | |
|---|---|---|---|---|---|---|---|---|---|
| | | | 1 | 2 | 3 | 4 | 5 | 6 | 7 |
| PDS | Com. Ex. | 16.65 | 11.5 | 11.7 | 11.0 | 10.0 | 11.9 | 11.6 | 9.7 |
| | Ex. 1 | 14.90 | 11.2 | 9.9 | 8.7 | 8.8 | 9.0 | 7.3 | 7.9 |
| | Ex. 2 | 12.66 | 7.8 | 8.9 | 8.4 | 7.0 | 6.5 | 6.4 | 5.8 |
| EPS | Com. Ex. | 12.25 | 6.4 | 5.6 | 6.1 | 6.4 | 4.6 | 6.4 | 6.3 |
| | Ex. 1 | 13.25 | 7.4 | 5.7 | 7.6 | 5.6 | 4.7 | 3.5 | 3.6 |
| | Ex. 2 | 8.84 | 4.6 | 7.3 | 7.1 | 4.0 | 4.2 | 1.0 | 2.8 |
| PDS+EPS | Com. Ex. | 28.90 | 17.9 | 17.3 | 17.1 | 16.4 | 16.5 | 18.0 | 16.0 |
| | Ex. 1 | 28.15 | 18.6 | 15.6 | 16.3 | 14.4 | 13.7 | 10.8 | 11.5 |
| | Ex. 2 | 21.50 | 12.4 | 16.2 | 15.5 | 11.0 | 10.7 | 10.4 | 8.6 |
| all symptoms | Com. Ex. | 33.75 | 20.2 | 20.1 | 20.4 | 19.1 | 19.6 | 22.0 | 19.8 |
| | Ex. 1 | 33.95 | 20.3 | 17.8 | 17.9 | 16.2 | 15.2 | 12.0 | 12.8 |
| | Ex. 2 | 25.22 | 14.7 | 20.6 | 17.4 | 12.6 | 12.3 | 12.0 | 9.9 |

| symptom | ingestion group | before ingestion | days after ingestion (days) | | | | | | |
|---|---|---|---|---|---|---|---|---|---|
| | | | 8 | 9 | 10 | 11 | 12 | 13 | 14 |
| PDS | Com. Ex. | 16.65 | 11.0 | 11.0 | 8.2 | 9.3 | 7.9 | 8.2 | 8.5 |
| | Ex. 1 | 14.90 | 8.4 | 8.6 | 8.1 | 7.4 | 6.1 | 7.3 | 6.1 |
| | Ex. 2 | 12.66 | 3.7 | 4.3 | 4.7 | 4.4 | 3.6 | 4.8 | 3.5 |
| EPS | Com. Ex. | 12.25 | 5.6 | 5.2 | 4.8 | 4.8 | 3.9 | 4.8 | 5.0 |
| | Ex. 1 | 13.25 | 3.9 | 5.0 | 5.1 | 3.7 | 4.2 | 4.5 | 2.9 |
| | Ex. 2 | 8.84 | 2.5 | 2.5 | 3.0 | 2.5 | 1.5 | 1.9 | 2.1 |
| PDS+EPS | Com. Ex. | 28.90 | 16.6 | 16.2 | 13.0 | 14.1 | 11.8 | 13.0 | 13.5 |
| | Ex. 1 | 28.15 | 12.3 | 13.6 | 13.2 | 11.1 | 10.3 | 11.8 | 9.0 |
| | Ex. 2 | 21.50 | 6.2 | 6.8 | 7.7 | 6.9 | 5.1 | 6.7 | 5.6 |
| all symptoms | Com. Ex. | 33.75 | 20.0 | 19.2 | 16.8 | 17.4 | 14.2 | 15.3 | 15.8 |
| | Ex. 1 | 33.95 | 13.5 | 15.4 | 14.9 | 12.0 | 11.3 | 12.7 | 10.3 |
| | Ex. 2 | 25.22 | 7.3 | 8.0 | 8.5 | 8.0 | 6.0 | 7.6 | 6.4 |

In Table 1, the granule ingestion group of Example 1 showed a tendency to improve each symptom of upper gastrointestinal tract after 2 days from the start of the ingestion for PDS symptoms, and 6 days from the start of the ingestion for EPS symptoms. The granule ingestion group of Example 2 showed a more remarkable improvement, and the total score of the PDS symptom and EPS symptom was clearly lower than that of the granule ingestion group of Comparative Example from 3 days to 14 days from the start of the ingestion. As for the PDS symptoms, the score at day 14 was 8.5 for the granule ingestion group of Comparative Example and 3.5 for the granule ingestion group of Example 2; 5 points lower on average. As for the EPS symptoms, the score at day 14 was 5.0 for the granule ingestion group of Comparative Example and 2.1 for the granule ingestion group of Example 2; 2.9 points lower on average. Thus, L-sodium glutamate and L-arginine hydrochloride showed an upper gastrointestinal symptom-improving tendency in a dose-dependent manner.

As is clear from the above, the granule ingestion groups of the Examples of the present invention showed a tendency to reduce upper gastrointestinal symptoms as compared to the granule ingestion group of the Comparative Example. Of the upper gastrointestinal symptoms, a particularly improving tendency was observed in items relating to the postprandial distress syndrome (PDS).

### Industrial Applicability

According to the present invention, an oral composition capable of effectively preventing or improving gastrointestinal dysfunction associated with functional degradation of the gastrointestinal tract can be provided. The oral composition of the present invention can be provided as an agent for the prophylaxis or improvement of gastrointestinal dysfunction, and can also be provided in a food or drink.

This application is based on a patent application No. 2009-062016 filed in Japan, the contents of which are incorporated in full herein.

## Claims

1. An oral composition comprising sodium glutamate and arginine hydrochloride at a molar ratio of 1:1.

2. The oral composition according to claim 1, wherein sodium glutamate and arginine hydrochloride are contained in a total amount of 0.05 g - 10 g.

3. The oral composition according to claim 1, wherein sodium glutamate and arginine hydrochloride are contained in a total amount of 0.1 g - 5 g.

4. The oral composition according to claim 1, wherein sodium glutamate and arginine hydrochloride are contained in a total amount of 0.3 g - 3 g.

5. The oral composition according to claim 1, wherein a total amount of sodium glutamate and arginine hydrochloride to be ingested per day for an adult is 0.3 g - 10 g.

6. The oral composition according to claim 1, wherein the amount to be ingested per day is taken in one to 3 portions.

7. The oral composition according to claim 1 for consecutive ingestion for 7 days or longer.

8. The oral composition according to claim 1, which is granule.

9. The oral composition according to claim 1, which is an oral agent for the prophylaxis or improvement of a functional gastrointestinal disorder.

10. The oral composition according to claim 9, wherein the functional gastrointestinal disorder is upper gastrointestinal dysfunction.

11. The oral composition according to claim 10, wherein the upper gastrointestinal dysfunction is functional dyspepsia.

12. A food or drink containing the oral composition according to claim 1.

13. A method for the prophylaxis or improvement of a functional gastrointestinal disorder, comprising orally administering the oral composition according to claim 1 in an amount sufficient for the prophylaxis or improvement of functional gastrointestinal disorder to a patient with a functional gastrointestinal disorder.

14. A method for the prophylaxis or improvement of an upper gastrointestinal dysfunction, comprising orally administering the oral composition according to claim 1 in an amount sufficient for the prophylaxis or improvement of upper gastrointestinal dysfunction to a patient with an upper gastrointestinal dysfunction.

15. A method for the prophylaxis or improvement of functional dyspepsia, comprising orally administering the oral composition according to claim 1 in an amount sufficient for the prophylaxis or improvement of functional dyspepsia to a patient with functional dyspepsia.

16. A commercial package comprising the oral composition according to any of claims 9 to 12 and a document stating that said oral composition can or should be used for the prophylaxis or improvement of a functional gastrointestinal disorder.
